# EUROPEAN PATENT SPECIFICATION

(11) **EP 0 710 472 B1**
(45) Date of publication and mention of the grant of the patent: **04.04.2001**
(21) Application number: 94203230.1
(22) Date of filing: 05.11.1994
(51) Int. Cl.: A61F 13/15

(54) **Breathable dual layer backsheet design for disposable absorbent articles**
Ausführung einer atmungsaktiven doppellagigen unteren Schicht für absorbierende Wegwerfartikel
Conception d'une feuille arrière à double couche perméable au gaz pour des articles absorbants jetables

(43) Date of publication of application: 08.05.1996
(73) Proprietor: THE PROCTER & GAMBLE COMPANY, Cincinnati, Ohio 45202 (US)
(72) Inventor: Depner, Michael, D-55130 Mainz (DE); Divo, Michael, D-61381 Friedrichsdorf (DE)
(74) Representative: Hirsch, Uwe Thomas

(56) References cited:
- EP-A- 0 040 447
- EP-A- 0 207 904
- EP-A- 0 257 280
- EP-A- 0 477 802
- EP-A- 0 495 212
- EP-A- 0 556 749
- WO-A-93/09741
- US-A- 3 881 489

## Description

### Field of the invention

The present invention relates to disposable absorbent articles such as baby diapers or sanitary napkins having a breathable yet liquid leakage retarding backsheet. In particular the backsheet comprises an inner and an outer layer which are essentially non-attached to each other. Both layers can be formed of the same or a different breathable material.

### Background of the invention

Disposable absorbent articles such as baby diapers, adult incontinence products, sanitary napkins and panty liners are well known in the art. These articles have a wearer facing side through which they typically absorb liquids discharged by the wearer. The liquid is stored in an absorbent structure. Liquid leakage from the article through the surface opposite the wearer facing side is usually prevented by incorporating a liquid impermeable backsheet on that side.

It is also well established in the art that a backsheet allowing gaseous fluid communication with the environment, usually referred to as breathability, is highly desirable. Breathability improves with the amount of air permeating through a backsheet. This amount is proportional to the open area (the sum of the area of all apertures) in the backsheet. Obviously too many and particularly too large apertures in the backsheet lead to compromising the liquid leakage prevention, which is the primary function of a backsheet.

Many suggestions how to provide breathable backsheets have been recorded in the art. Numeral attempts of combining the mutual contradicting features of gas permeability and liquid impermeability have been documented in patents and patent applications. However the lack of commercially available and truly breathable disposable absorbent articles indicates that the technology so far suggested has not provided an all around satisfactory result for the desired technical requirements at commercially acceptable condition. More often than not satisfaction of one desired feature went to such an extreme that the respective other feature was not properly satisfied any longer.

For example sanitary napkins with very high breathability at the cost of frequent liquid leakage (leading to soiling of the undergarments of a wearer) cannot be considered satisfactory. On the other hand satisfying the liquid leakage problem properly usually resulted in almost impermeable, that is non-breathable, backsheets.

Good progress has been made in the field of formed films having directional liquid transport wherein liquid transport over a certain pressure drop across a formed film is better in one direction versus the other. These so called one way formed film materials have found wide usage as liquid permeable topsheets after being treated with surfactant in the field of sanitary napkins and panty liners, but also for diapers and incontinence products. Alternative topsheets of fibrous fabric polymers typically have no directional liquid transport. They are also treated with surfactant but have an intrinsic hydrophobic behaviour. Use of topsheet materials with adaptation to be hydrophobic and reducing pore size and/or total open area has also been proposed for breathable backsheets.

Combinations of breathable and liquid permeable sheets in order to provide a certain liquid impermeability while satisfying the desire for breathable backsheets have already been suggested for example in US 3,881,489. In this disclosure a breathable backsheet is provided by confining an outer layer of formed film material having surface aberrations with apertures therein and an inner layer of a paper tissue having a high void volume and having been made hydrophobic by impregnating it with a paraffin wax.
This document however includes instruction to substantially attach the layers across their entire interface.

Other prior art attempts to provide breathable backsheet assemblies comprising more than one layer are e. g. documented in US 4,341,216, EP-A-109 126 or EP-A-203 821. Neither of these disclosures provides constructions of breathable backsheets similar to the present invention.

Single layer breathable backsheets are known for example from GB-A-2184391, GB-A-2184390, GB-A-2184389, US 4,591,523, US 4,839,216 or EP 156471.

In EP 477802 an absorbent article comprising a liquid impermeable, optionally vapour permeable backsheet structure is described, wherein the backsheet has a soft and cloth-like outer texture. The backsheet structure comprises a single layer liquid impermeable, optionally vapour permeable inner baffle, e.g. a microporous polymer film, and a nonwoven outer layer substantially not attached to the baffle layer, which is intended to provide the backsheet structure with said cloth-like texture. It has now been found that combining two breathable layers of materials and maintaining the two layers substantially unattached to each other provides a particularly desirable breathable backsheet. Backsheets according to this construction can have exceptionally good breathability due to a large open area and the combination of two layers provides the desired liquid leakage retarding function. Importantly it is now possible to replace a single breathable layer by 2 breathable layers having the same total material consumption, essentially the same breathability but better leakage prevention for being substantially unattached to each other. Therefore the present invention provides an non-leaking breathable backsheet construction and absorbent articles comprising this backsheet.

It is therefore an objective of the present invention to provide absorbent articles in particular sanitary napkins or panty liners having good breathability while simultaneously retarding liquid leakage through that backsheet to such an extent that the user of such products does not experience a recognisable difference between a liquid impermeable backsheet and the breathable backsheet according to the present invention.

An additional objective satisfied by the present invention is to provide breathable backsheets at parity material consumption to known breathable backsheets which have reduced leakage performance without reduced breathability.

### Cross reference

Another patent application is being filed on the same date as this application. It is entitled "Breathable backsheet design for disposable absorbent articles" by M. Depner and M. Divo and also assigned to "The Procter and Gamble Company".

### Description of the invention

The present invention relates to breathable absorbent articles such as baby diapers, adult incontinence products, sanitary napkins or panty liners. Typically such products have a topsheet, a backsheet and an absorbent core between the topsheet and the backsheet. The core defines the core-area of the article which is the area coextensive with the absorbent core. The area outside the core-area is the peripheral area. The articles according to the present invention have a breathable backsheet comprising at least two breathable layers. Both layers are substantially unattached to each other the core-area, i.e. they are at least 50%, preferably 90%, most preferably fully un-attached over the core-area.

The breathable layers of the backsheet can be so as to replace a single breathable backsheet layer by at least two layers of the same material with a total material consumption equal to the replaced single breathable layer. In particular two breathable layers forming together the breathable backsheet are preferred. The two layers can however preferably have a different basis weight: one being stronger, i.e. having a higher basis weight than the other. The stronger one would be placed on the outside of the absorbent article.

In an embodiment of the present invention the breathable backsheet comprises a hydrophobic, gas-permeable fibrous fabric layer composed of polymeric fibres. In the present invention the breathable backsheet comprises a hydrophobic, gas-permeable apertured polymeric film. This film has a first liquid transport direction and a second liquid transport direction opposite the first liquid transport direction and is oriented such that the first direction is from the backsheet towards the absorbent core. This directional apertured film allows a liquid transport in the first liquid transport direction which is larger than the liquid transport in the second liquid transport direction under an identical pressure drop across said apertured film. Preferably the film comprises funnel shaped apertures wherein the direction from the larger funnel opening towards the smaller funnel opening is parallel to the first liquid transport direction.

It is preferred that the breathable backsheet of the absorbent article provides a threshold pressure below which a test liquid does not permeate in the second liquid transport direction through the backsheet, thereby providing a "zero leakage" threshold. This threshold will be dependent on the usage circumstances of the product, e. g. babies will not provide a pressure onto their diaper when sitting as large as women onto their panty liners when bicycling.

For sanitary napkins or panty liners the respective pressure threshold has been found to be equivalent to 45 g/cm² (i. e. 4414.5 Pa) according to the test protocol disclosed in the example below. The test liquid used to identify test leakage comprises a saline solution consisting of 2 g urea, 0.9 g sodium chloride, 0.06 g calcium chloride and 0.11 g hydrated magnesium sulphate in 100 ml distilled water. This solution is adjusted for surface tension by adding surfactant to better simulate bodily discharges other than urine. The reduced surface tension of the solution 29 +/-1 mN/m while the saline solution without surfactant has a surface tension of more than 60 mN/m.

The fibrous fabric layer useful in the present invention preferably has a basis weight of 10 to 100 g/m² preferably 15 to 30 g/m². The fibres can be made of any hydrophobic polymeric material usual in the art of making fibrous fabric layers. Depending on the circumstances of the ultimate use and manufacturing of the breathable absorbent article fibres of polyethylene, polypropylene, polyester, polyacetat or combinations thereof (intra- and inter-fibres combinations) have been found useful.

The fibres are preferably spunbonded, carded or melt blown. The fabric layer most preferably comprises a matrix of spunbonded fibres covered on one or both sides with meltblown fibres but can also be provided by any other typical technology used in the art. If two or more fibrous fabric layers of different basis weight are used it is most preferred to have the one with the highest basis weight on the outside of the absorbent article.

The apertured film useful for embodiments of the present invention can be any of those well known in the art. This includes in particular, but is not limited to those films disclosed in U.S. 3,929,135, U.S. 4,151,240, U.S. 4,319,868, U.S. 4,324,246, U.S. 4,342,314 , U.S. 4,591,523 and U.S. 4,609,518, U.S. 4,629,643, U.S.5,158,819, U.S. 4,772,444.

The apertured film comprised in the inner layer of the breathable backsheet preferably has funnel shaped apertures similar to those described e. g. in US 3,929,135. The apertures maybe circular or non-circular but have a cross sectional dimension at one end of the funnel which is wider than the opening at the other end of the funnel. The direction from the larger funnel opening towards the smaller opening is of course parallel to the first liquid transport direction. The open area of the apertured film is typically more than 5 %, preferably in the range of 10 % to 35 % of the total film surface. The apertured films can be made of any material typical in the art but preferably is made of a polymer similar to those used for the fibrous fabric layer.

The minimum hydraulic diameter of the apertures in the film should be as small as possible while still providing sufficient gas permeability without hydraulic blockage of the apertures. A hydraulic diameter of as little as 0.1 mm, preferably 0.2 to 0.7 mm has been found possible in the context of the present invention.

Hydraulic diameter for non circular apertures is the diameter that a circular aperture with the same cross section would have. Diameter is always determined in the plane of smallest cross section of an aperture.

In principle the present invention of substantially un-attached layers of breathable material as backsheet to improve the soiling through the breathable layer is useful for any breathable layer material known and can be applied in any absorbent article. Of course the layers of the breathable backsheet need to be combined somewhere to create the breathable backsheet according to the present invention. Otherwise the outer layers of the backsheet would simply fall off. However it is important that the layers are as little attached to each other at least across the core-area of absorbent article in order to minimise leakage and soiling through the backsheet. This combining can for example be provided in the periphery outside the area coextensive with the absorbent core or in a pattern of lines or dots across the whole area coextensive with the absorbent core which pattern does not cover any significant area itself.

The present invention as indicated above can be used beneficially in the context of many different absorbent articles. However sanitary napkins and especially thin panty liners are particularly susceptible to the present invention. Sanitary napkins or panty liners having a thickness of 3 mm or less and preferably 2 mm or less benefit especially well from the breathable backsheet of the present invention.

The disposable absorbent articles according to the present invention can have all those other features and parts which are typical for products in the context of their intended use. They comprise typically the absorbent core which can be a fluffy fibrous absorbent core comprising also hydrogel particles if desired, laminated tissues with or without particulate materials including hydrogel particles or odour control particles. The absorbent core fibres can be any of those known in the art including cellulose fibres or polymeric fibres rendered absorbent or even non-absorbent matrix fibres. Also tissues of sufficient basis weight and absorbency can be used as the absorbent structure according to the present invention. E.g. the tissue used in the method described below to measure wet-through can be used in the context panty liners.

Also a topsheet or wearer contacting layer through which the liquids to be absorbed penetrate to the absorbent structure is typically incorporated in articles according to the present invention. The topsheet or wearer contacting layer can be provided by any of those materials and techniques known in the art including formed films and non-woven fibrous fabrics similar to those described herein above.

### Examples

For testing the following examples the wet-through method as described below was used. It follows conceptually the wet-through method disclosed in US 3,881,489 but relates to the backsheet wet- through prevention ability.

### Test preparation

The breathable backsheet to be analysed is provided with a generally hydrophilic, test liquid absorbing tissue on the inside surface. The tissue is placed without exerting pressure on the backsheet. Then samples of 5 cm times 5 cm are cut and compressed under a load of 40 g/cm² (or 1.0 kg per sample). Not required for the test but desirable for easy operation of the test is an additional layer of non woven fabric, or formed film on top of the absorbent tissue.

For repeatability the samples are kept for at least 4 hours at test conditions. Test conditions are 23°C and 50% relative humidity.
The test liquid is prepared by mixing 100 ml of distilled water, 2 g urea, 0,9 g NaCI, 0,11 g MgSO₄ x 7 H₂O and 0.06 g CaCl₂. The test liquid then is adjusted to a surface tension of 29 +/- 1 mN/m to resemble particularly the condition exhibited by women with vaginal discharge. To resemble other discharges the surface tension can be kept unaltered at about 60 mN/m.

### Test materials

Typical test materials are e. g. a tissue designated 609912, of 99 g/m² obtainable from the Merfin Hygienic Products company, in Delta, B.C., Canada. Generally the tissue should be not thicker than 1 mm.

The blotter paper can be any good absorbent blotter paper commercially available. For the tests below blotter paper designated Absorbent bianco No 30 (220 g/m²) of Cartiera, Favini in 36028 Rossano Veneto (VC), Italy was used. The blotter paper can be cut into larger areas than the 5 cm x 5 cm sample size.

The desirable non-woven or formed film shall not be absorbent and shall have no surface residue wash-off which would alter the test liquid composition. This can be achieved by washing the material with test liquid and drying it prior to it's use.

### Test liquid quantity

The test liquid quantity to be used in the test is determined as the amount necessary to saturate the absorbent tissue. This can be determined by preparing tissue samples of 5 cm x 5 cm, preferably larger but with a known surface area, of the tissue to be used in the wet-through test. The weight of the tissue sample is determined.

The tissue is then placed on a nylon net having rectangular and diagonal running threads stretched inside a frame. The net forms rectangles of 5 cm x 4,5 cm which are split into 4 equal parts by threads connecting the corners diagonally. The frame is preferably of a water repellent material such as a hydrophobic polymer.

The net together with the tissue samples are then immersed in destilled water of 23° C (+/- 1° C) for 30 seconds (+/- 3 seconds). Then the net together with the tissue is left to drain /drip in a horizontal position only under gravitational forces for 120 seconds (+/- 5 seconds). Then the weight of the tissue together with the absorbed water is measured. The scales for the weight measurements should be accurate to 0.1 gram.

The amount of absorbed water is calculated (wet weight minus dry weight) and divided by the surface area of the tissue sample. This value is multiplied by the surface area of 25 cm² of the wet-through test samples (5 cm x 5 cm) to result in the test liquid quantity to be used. Statistical analysis should be used to ensure an accuracy of +/- 10% of the test liquid quantity value within a +/- 3 Sigma range of the test series (adjusted for surface area of 25 cm².

### Test method to determine wet-through at defined load

The samples are placed on an absorbent blotter paper with the breathable backsheet adjacent the blotter paper. The size of the blotter paper should be larger than the test sample size. The blotter paper is conditioned as the test samples are and its weight prior to the test is taken.

Then the defined test liquid quantity is placed in the centre of the sample. The flow rate when placing the liquid should be about 1 ml/10 sec., i. e. the test liquid should be placed onto the sample in about 20 sec. for 2 ml loadings. After 2 minutes a load of 40 g/cm² is applied to the wet sample for 15 seconds. Typically the minimum load is 20 g/cm² or 0.5 kg per sample, however for simulating a stress condition a load of 40g/cm² is applied. After removal of the load the blotter paper is checked for wet-through.

If there is wet-through the occurrence is noted as qualitative result or the weight of the blotter paper with the leaked absorbent liquid which has been absorbed is measured. The difference of the second measurement to the original dry weight of the blotter paper is the amount of wet-through which provides a quantitative result.

Care must be taken to not introduce side leakage e.g. due to an uneven liquid migration from the centre of the sample. The samples must be stored horizontally during the test. If side leakage occurs anyway at the edges of the sample and wet trough is observed the quantitative amount of wet-through outside the sample area is to be disregarded. If all test samples have side edge leakage without wet-through occurring a different tissue should be used to confirm the test result. If the result is consistent then the qualitative and quantitative results are no wet-through.

### Results

At least 10 samples should be measured and averaged. Statistical analysis should be used to confirm that the average result is statistically correct within a 95% confidence interval.

### Test method to determine wet-through threshold load

This method is identical to the test method at constant load except that the load starting from 20 g/cm2 is gradually increased by increments of 5 g/cm² (125 g per sample). The measurement of wet-through quantity is not required but the test person needs to determine the lowest load at which wet-through occurs. The threshold load is defined as the lowest load at which wet-through occurs minus 5 g/cm². If wet-through occurs at 20 g/cm² already the breathable backsheet is reported to be leaking without threshold load.

### Test Products

Backsheets according to the present invention were constructed from the following raw material:
- non-woven fabric of 28 g/m² having a spunbonded layer of 14 g/m² and a melt blown layer of 14 g/m² obtainable from Corovin GmbH, Peine, Germany under the designation MD 2000.
- polyethylene formed film according to US 3,929,135 obtainable from Tredegar Film Products B.V., Kerkrade, The Netherlands. The film has circular funnel shaped apertures with an open area of 19%, an embossed thickness of 0.48 mm (funnel height) and an aperture diameter of 0,465 mm.

The backsheet according to the invention is prepared by joining the film with the funnels pointing towards the absorbent structure (for this testing the absorbent structure was the 99 g/m² Merfin tissue described supra) and the fabric with the melt blown layer being oriented to become the inter face between formed film and fabric.

In an example according to the present invention the fabric and the film are overlaying each other without attaching the two layers at all. As reference a comparison of adhesively attached and unattached leakage above the threshold pressure has been measured as well. The adhesive attachment for the comparison sample was spiral adhesive applied in 3 spirals of 25mm diameter at a basis weight of 6 g/m² covering a total area of 1% to 2% The adhesive used is designated H2128 available from Findley, Roosendaal, The Netherlands. The test liquid was used at 2 ml per sample.

### Threshold pressure test with reduced surface tension test liquid:

| | |
|---|---|
| First leakage observed at | 50 g/cm² |
| Threshold pressure | 45 g/cm² |

### Wet/through test

Wet/through qualitative test were conducted above 50 g/cm² with test liquid having a surface tension 29 mN/m. Results are given in percent of test liquid quantity. The results provide a valuable display of the benefit of maintaining the breathable structure un-attached.

| Sample: | un-attached layers | attached layers |
|---|---|---|
| leakage through the backsheet | 1.5% | 18,5% |
| index | 8.1 | 100 |

## Claims

1. Breathable absorbent article comprising a topsheet, a breathable backsheet and an absorbent core between said topsheet and said backsheet, said article having a core-area which is coextensive with said absorbent core and a peripheral area which is coextensive with said article excluding said core-area, said breathable backsheet comprising at least two separate, breathable layers, said breathable absorbent article characterized in that : said layers are un-attached across at least 50% of said core-area, wherein said breathable backsheet comprises a hydrophobic, gas-permeable apertured polymeric film,
wherein said film has a first liquid transport direction and a second liquid transport direction opposite said first liquid transport direction and being oriented such that said first direction is from said backsheet towards said absorbent core, said apertured film allowing a liquid transport in said first liquid transport direction which is larger than the liquid transport in said second liquid transport direction under an identical pressure drop across said apertured film,

2. Breathable absorbent article according to claim 1, wherein said apertured film comprises funnel shaped apertures wherein the direction from the larger funnel opening towards the smaller funnel opening is parallel to said first liquid transport direction.

3. Breathable absorbent article according to claims 1 or 2 wherein said breathable layers are un-attached across at least 90% of said core-area.

4. Breathable absorbent article according to claim 3 wherein said breathable layers are un-attached across all of said core-area.

5. Breathable absorbent article according to any of the preceding claims wherein said breathable backsheet has no liquid transport in a direction away from said absorbent core under a pressure of 45 g/cm² for an aqueous saline solution consisting of 100 ml distilled water, 2 g urea, 0.9 5 g NaCI, 0.11 g MgSo₄ x 7H₂O, 0.06 g CaCl₂, the saline solution is adjusted with surfactant to 29⁺/- 1 mN/m.

6. Breathable absorbent article according to any of the preceding claims wherein said breathable backsheet consists of two layers.

7. Breathable absorbent article according to any of the preceding claims wherein said breathable backsheet 20 comprises a hydrophobic, gas-permeable fibrous fabric layer composed of polymeric fibres.

8. Breathable absorbent article according to any of the claims wherein all of said breathable layers are identical.

## Patentansprüche

1. Atmungsaktiver absorbierender Artikel, der ein Deckblatt, ein atmungsaktives Rückenblatt und einen absorbierenden Kern zwischen dem genannten Deckblatt und dem genannten Rückenblatt umfaßt, welcher genannte Artikel einen Kernbereich, der mit dem genannten absorbierenden Kern coextensiv ist, und einen peripheren Bereich, der mit dem genannten Artikel, mit Ausnahme des genannten Kernbereichs, coextensiv ist, hat, welches genannte atmungsaktive Rückenblatt mindestens zwei getrennte atmungsaktive Schichten umfaßt,
wobei der genannte atmungsaktive absorbierende Artikel dadurch gekennzeichnet ist, daß die genannten Schichten über mindestens 50 % des genannten Kernbereichs hinweg unverbunden sind, wobei das genannte atmungsaktive Rückenblatt eine hydrophobe, gasdurchlässige, mit Öffnungen versehene polymere Folie umfaßt, welche genannte Folie eine erste Flüssigkeitstransportrichtung und, der genannten ersten Flüssigkeitstransportrichtung entgegengesetzt, eine zweite Flüssigkeitstransportrichtung aufweist und so orientiert ist, daß die genannte erste Richtung von dem genannten Rückenblatt in Richtung zu dem genannten absorbierenden Kern liegt, wobei die genannte mit Öffnungen versehene Folie einen Flüssigkeitstransport in der genannten ersten Flüssigkeitstransportrichtung erlaubt, der unter einem identischen Druckabfall über die genannte mit Öffnungen versehene Folie hin größer ist als der Flüssigkeitstransport in der genannten zweiten Flüssigkeitstransportrichtung.

2. Atmungsaktiver absorbierender Artikel nach Anspruch 1, in welchem die genannte mit Öffnungen versehene Folie trichterförmige Öffnungen aufweist, wobei die Richtung von der größeren Trichteröffnung zu der kleineren Trichteröffnung parallel zu der genannten ersten Flüssigkeitstransportrichtung liegt.

3. Atmungsaktiver absorbierender Artikel nach Anspruch 1 oder 2, in welchem die genannten atmungsaktiven Schichten über mindestens 90 % des genannten Kernbereichs hin unverbunden sind.

4. Atmungsaktiver absorbierender Artikel nach Anspruch 3, in welchem die genannten atmungsaktiven Schichten über den gesamten genannten Kernbereich hin unverbunden sind.

5. Atmungsaktiver absorbierender Artikel nach einem der vorhergehenden Ansprüche, in welchem das genannte atmungsaktive Rückenblatt in einer Richtung weg von dem genannten absorbierenden Kern unter einem Druck von 45 g/cm² keinen Flüssigkeitstransport für eine wässerige Salzlösung hat, die aus 100 ml destilliertem Wasser, 2 g Harnstoff, 0,9 g NaCl, 0,11 g MgSO₄ x 7H₂O, 0,06 g CaCl₂ besteht und mit Tensid auf 29 +/- 1 mN/m eingestellt ist.

6. Atmungsaktiver absorbierender Artikel nach einem der vorhergehenden Ansprüche, in welchem das genannte atmungsaktive Rückenblatt aus zwei Schichten besteht.

7. Atmungsaktiver absorbierender Artikel nach einem der vorhergehenden Ansprüche, worin das genannte atmungsaktive Rückenblatt eine hydrophobe, gasdurchlässige faserige Textilmaterialschichte aus polymeren Fasern umfaßt.

8. Atmungsaktiver absorbierender Artikel nach einem der vorhergehenden Ansprüche, in welchem alle genannten atmungsaktiven Schichten identisch sind.

## Revendications

1. Article absorbant pouvant respirer comprenant une feuille de dessus, une feuille de fond pouvant respirer et une âme absorbante entre ladite feuille de dessus et ladite feuille de fond, ledit article présentant une zone d'âme qui coïncide approximativement avec ladite âme absorbante et une zone périphérique qui coïncide approximativement avec ledit article à l'exclusion de ladite zone d'âme, ladite feuille de fond pouvant respirer comprenant au moins deux couches pouvant respirer, séparées, ledit article absorbant pouvant respirer étant caractérisé en ce que lesdites couches ne sont pas fixées sur au moins 50% de ladite zone d'âme, dans lequel ladite couche de fond pouvant respirer comprend un film polymère perforé perméable aux gaz, hydrophobe, dans lequel ledit film a une première direction de transport de liquide et a une deuxième direction de transport de liquide opposée à ladite première direction de transport de liquide et étant orienté de manière que ladite première direction aille de ladite feuille de fond vers ladite âme absorbante, ledit film perforé permettant un transport de liquide dans ladite première direction de transport de liquide, qui est plus important que le transport de liquide dans ladite deuxième direction de transport de liquide sous l'effet d'une chute de pression identique à travers ledit film perforé.

2. Article absorbant pouvant respirer selon la revendication 1, dans lequel ledit film perforé comprend des orifices en forme d'entonnoir, dans lequel la direction allant de la plus grande ouverture d'entonnoir vers la plus petite ouverture d'entonnoir est parallèle à ladite première direction de transport de liquide.

3. Article absorbant pouvant respirer selon les revendications 1 ou 2, dans lequel lesdites couches pouvant respirer ne sont pas fixées sur au moins 90% de ladite zone d'âme.

4. Article absorbant pouvant respirer selon la revendication 3, dans lequel lesdites couches pouvant respirer ne sont pas fixées sur toute ladite zone d'âme.

5. Article absorbant pouvant respirer selon l'une quelconque des revendications précédentes, dans lequel ladite feuille de fond pouvant respirer n'a pas de transport de liquide dans une direction à distance de ladite âme absorbante sous une pression de 45g/cm² pour une solution aqueuse saline constituée de 100 ml d'eau distillée, 2 g d'urée, 0,9 g de NaCl, 0,11 g de MgS0₄ x 7H₂0, 0,06 g de CaCl₂, la solution saline étant ajustée avec un agent tensioactif à 29 +/- 1 mN/m.

6. Article absorbant pouvant respirer selon l'une quelconque des revendications précédentes, dans lequel ladite feuille de fond pouvant respirer est constituée de deux couches.

7. Article absorbant pouvant respirer selon l'une quelconque des revendications précédentes, dans lequel ladite feuille de fond pouvant respirer comprend une couche de tissu fibreux peméable aux gaz, hydrophobe, composée de fibres polymères.

8. Article absorbant pouvant respirer selon l'une quelconque des revendications précédentes, dans lequel toutes lesdites couches pouvant respirer sont identiques.
